Europäisches Patentamt

European Patent Office

Office européen des brevets

(11) Publication number: **0 603 405 A1**

## EUROPEAN PATENT APPLICATION
### published in accordance with Art. 158(3) EPC

(21) Application number: **93910373.5**

(22) Date of filing: **21.05.93**

(86) International application number:
**PCT/JP93/00675**

(87) International publication number:
**WO 93/24129 (09.12.93 93/29)**

(51) Int. Cl.⁵: **A61K 31/585**, A61K 47/14, A61K 47/16

(30) Priority: **25.05.92 JP 132275/92**

(43) Date of publication of application:
**29.06.94 Bulletin 94/26**

(84) Designated Contracting States:
**DE ES FR GB IT**

(71) Applicant: **TOKO YAKUHIN KOGYO KABUSHIKI KAISHA**
**14-25, Naniwa-cho,**
**Kita-ku**
**Osaka-shi, Osaka 530(JP)**

(72) Inventor: **KAMISHITA, Takuzo**
**12-27, Tonda-cho 6-chome**
**Takatsuki-shi, Osaka 569(JP)**
Inventor: **MIYAZAKI, Takashi**
**41, Kamisho,**
**Kamiichi-machi**
**Nakaniikawa-gun, Toyama 930-03(JP)**
Inventor: **MORISHITA, Chieko**
**1-2-2-50, Yokohoonji,**
**Kamiichi-machi**
**Nakaniikawa-gun, Toyama 930-03(JP)**

(74) Representative: **Weisert, Annekäte, Dipl.-Ing.**
**Dr.-Ing. et al**
**Patentanwälte**
**Kraus Weisert & Partner**
**Thomas-Wimmer-Ring 15**
**D-80539 München (DE)**

(54) COMPOSITION FOR TREATING ACNE VULGARIS.

㊼ A composition for treating acne vulgaris, which comprises spironolactone as the active ingredient and an ordinary base for external preparations and further contains crotamiton and/or glycol monosalicylate as a solvent for the spironolactone.

## Technical Field

This invention relates to a pharmaceutical composition for the treatment of acne vulgaris comprising as an active ingredient spironolactone. More particularly, it relates to a pharmaceutical composition for the treatment of acne vulgaris by percutaneous administration of spironolactone which has usually been used as an antihypertensive agent due to its diuretic activity by oral administration.

## Background Art

Acne vulgaris is one of the frequently occurring dermatologic diseases, and the occurring mechanism is roughly classified into two bases, one being due to sebiparous hypersecretion induced by accelerated function of sebaceous gland associated with genetic background or sexual hormones, another one being due to arctation of hair opening by hyperkeratosis. The symptom will be changed in the inflammatory aspect due to the irritation with free fatty acids produced by the decomposition of triglycerides contained in the sebum with a lipase of an indigenous bacteria on the skin (Bacillus acne), and thereby, wheal, pustula, induration, abscess, or the like are induced.

The acne vulgaris has usually been treated by oral administration of vitamins or hormones such as vitamin A or vitamin $B_2$, while it depends on the symptoms, or when comedo appeares owing to the arctation of hair opening, by percutaneous administration of a sulfur agent having a keratolytic activity, and further when a deep red color wheal appears, by oral administration of tetracycline antibiotics, or occasionally steroid compounds.

Spironolactone has an antihypertensive effect due to the diuretic activity and hence has usually been used as an antihypertensive agent, but on the other hand, it has been known that it shows also an inhibitory activity on sebiparous by percutaneous administration and hence has been tried to prepare as a dermatologic drug. For instance, Messina has reported a pharmaceutical composition for the treatment of acne vulgaris comprising 0.1 to 10 % by weight of spironolactone, 10 to 25 % by weight of a mixed ester of a saturated higher fatty acid with polyethylene glycol and polyoxyethylene glycol, 2 to 10 % by weight of a saturated polyoxyethylene glycol glyceride, and 60 to 75 % by weight of water (cf. U.S. Patent 4,543,351).

In order to exhibit the effect of spironolactone by applying the spironolactone-containing composition to the skin, it is necessary to make the drug sufficiently absorbed through the skin. According to the study by the present inventors, it is necessary to incorporate the spironolactone into the substrate in the state of being dissolved in a degree as high as possible in order to achieve said purpose. However, spironolactone is hardly soluble in the conventional substrate components for the dermatologic drug such as water, vaseline, higher alcohols, vegetable oils, polyvalent alcohols, fatty acid esters and hence, it is very difficult to solubilize it in the substrate. It may be possible to dissolve it in the substrate by using a large amount of a lower alcohol such as ethanol, isopropanol, etc., but it is not preferable to use such a large amount of lower alcohols in view of the irritation thereof on the skin.

## Disclosure of the Invention

The present inventors have intensively studied as to various solvents and solubilizers in order to enhance the solubility of spironolactone in the pharmaceutical composition, and as a result, it has unexpectedly been found that spironolactone dissolves very well in crotamiton and glycol monosalicylate, and that a dermatologic drug prepared by using said solution can exhibit the desired effects very well, and hence, the composition is excellent as an agent for the treatment of acne vulgaris. Thus, the present invention has been accomplished.

The present invention provides a pharmaceutical composition for the treatment of acne vulgaris which comprises spironolactone as an active ingredient and either one or both of crotamiton and glycol monosalicylate as a solvent.

The pharmaceutical composition for the treatment of acne vulgaris of the present invention contains as the active ingredient spironolactone of 0.1 to 10 parts by weight based on the total weight of the composition and crotamiton and/or glycol monosalicylate as the solvent as well as appropriate substrate components for the conventional dermatologic drugs depending on the desired preparation form.

The solvent for spironolactone, crotamiton and/or glycol monosalicylate to be contained in the composition for the treatment of acne vulgaris of the present invention is contained in an amount sufficient to dissolve spironolactone, i.e. 0.1 to 10 parts by weight per one part by weight of spironolactone.

When a single of the solvents is used, it requires occassionally a very large amount thereof in order to dissolve spironolactone well, or it dissolves insufficiently spironolactone, and in such a case both of the solvents may be used.

The pharmaceutical composition for the treatment of acne vulgaris of the present invention may be formulated in any conventional preparation for dermatologic use, such as ointment, cream, gel, gel creame, lotion, solution, and the like. These preparations may be prepared by a conventional method by dissolving the active spironolactone in crotamiton and/or glycol monosalicylate and incorporating it into a conventional substrate for dermatologic drug.

For example, in the preparation of an ointment, vaseline, higher alcohols, beeswax, vegetable oils, polyethylene glycol, etc. are used. In the preparation of a cream preparation, fats and oils, waxes, higher fatty acids, higher alcohols, fatty acid esters, purified water, emulsifying agents etc. are used. In the preparation of a gel preparation, there are used a gelling substrate selected from a combination of a carboxyvinyl polymer and a water-soluble basic compound (e.g. alkali metal hydroxides, alkanolamines, etc.), hydroxypropyl cellulose, hydroxymethylpropyl cellulose, and the like.

In the preparation of a gel cream preparation, an emulsifying agent (preferably nonionic surfactants), an oily substance (e.g. liquid paraffin), etc. are used in addition to the above gelling substrate.

The substrate to be used for the gel preparation and gel cream preparation may also include an aqueous solution of carboxyvinyl polymer incorporated with one or more amino acids selected from alanine, valine, isoleucine, serine, cysteine, ornithine, lysine, arginine, histidine, proline, threonine, and methionine for increasing the viscosity of the solution, as disclosed in Japanese Patent Application No. 62-007044 of the present inventors.

In the preparation of a lotion, lower alcohols (e.g. ethanol, isopropanol, etc.) and polyvalent alcohols (e.g. propylene glycol, 1,3-butylene glycol, etc.) are used. In the preparation of a solution preparation, lower alcohols (e.g. ethanol, iropropanol, etc.), purified water, the above-mentioned polyvalent alcohols are used.

In addition to the above-mentioned ingredients, paraffines, squalane, lanolin, cholesterol esters, higher fatty acid esters, and the like may optionally be used. Moreover, any antioxidants such as BHA, BHT, propyl gallate, pyrogallol, tocopherol, etc. may also be incorporated.

In addition to the above-mentioned preparations and components, there may optionally be used any other conventional formulations for dermatologic drug or be incorporated with any other additives. Besides, the dermatologic preparations may be prepared by any other conventional methods or formulations. By the way, spironolactone may be decomposed at below pH 3 or higher than pH 7, and hence, a pH adjustor (for example, organic acids such as acetic acid, citric acid, or alkalis such as sodium hydroxide, triethanolamine) may preferably be used to adjust the pH value of the composition in the range of pH 4 to pH 6.

Best Mode for Carrying Out the Invention

The composition of the present invention is illustrated by the following Experiments and Examples.

Experiment 1

The solubility of spironolactone was tested in various solvents. The results are shown in Table 1. As is clear from the data shown in Table 1, among the various solvents, crotamiton and glycol monosalicylate showed specifically higher solubilizing effect for spironolactone.

Table 1

| Solvents | Amount of solvent for completely dissolving 1 g of spironolactone (g) |
|---|---|
| Crotamiton | 5 |
| Glycol monosalicylate | 6 |
| Peppermint oil | 12 |
| Methyl salicylate | 15 |
| Fats and Oils: sesame oil | >1000 |
| Wax: beeswax | >1000 |
| Hydrocarbons: | |
| Liquid paraffin | >5000 |
| Squalane | >150 |
| Vaseline | >1000 |
| Lower alcohols: | |
| Ethanol | 36 |
| Isopropanol | 150 |
| Methanol | 145 |
| Higher alcohols: | |
| 2-Octyldodecanol | 200 |
| Polyvalent alcohols: | |
| 1,3-Butylene glycol | 70 |
| Polyethylene glycol 400 | 40 |
| Esters: | |
| Isopropyl myristate | 150 |
| Diisopropyl adipate | 20 |
| Octadodecyl myristate | >300 |
| Isopropyl palmitate | 200 |
| Butyl stearate | 250 |
| Diethyl sebacate | 20 |
| Glyceryl tricaprate | 80 |
| Propylene glycol didecanate | 70 |
| Purified water | >4000 |

Experiment 2

In order to study the effect of crotamiton and glycol monosalicylate in a gel preparation, there were prepared the gel preparations having the formulations as shown in Table 2, and the solubility of spironolactone therein was tested. As a result, as is shown in Table 2, in the gel preparations (No. 7 to 11) incorporated with crotamiton or glycol monosalicylate, when they were kept under a severe condition of 5°C, spironolactone precipitated in a certain prepartions during the period of one week to one month, but when they were kept at room temperature, any precipitation of crystal was not observed even after kept for 3 months. On the contrary, in the preparations wherein these solvents were not used (No. 1 to 6), the preparation could not be prepared, or even if it could, the precipitation appeared within one week even when kept at room temperature, and hence, these preparations were not suitable.

Table 2

| Components | No. 1 | 2 | 3 | 4 |
|---|---|---|---|---|
| Spironolactone | 5 | 5 | 5 | 5 |
| Ethanol | 75 | 25 | | |
| Isopropanol | | 50 | | 50 |
| 1,3-Butyleneglycol | | | 45 | |
| Polyethylene glycol 400 | | | 40 | 25 |
| Isopropyl myristate | | | | |
| Diisopropyl adipate | | | | |
| Carboxyvinyl polymer | 1.3 | 1.3 | 1.3 | 1.3 |
| Hydroxypropylmethyl cellulose | 1.0 | 1.0 | | 1.0 |
| Triethanolamine | | | 0.05 | |
| Diisopropanolamine | 0.05 | 0.05 | | 0.05 |
| Crotamiton | | | | |
| Glycol monosalicylate | | | | |
| Purified water | 17.65 | 17.65 | 8.65 | 17.65 |
| Evaluation | A | A | B | C |

A: It could not be prepared because spironolactone was not dissolved; B: Crystals were precipitated immediately after the preparation; C: After being kept at room temperature for one day, crystals were precipitated.

Table 2 (Continue)

| Components | No. 5 | 6 | 7 | 8 |
|---|---|---|---|---|
| Spironolactone | 5 | 5 | 5 | 5 |
| Ethanol | 20 | 20 | 10 | 10 |
| Isopropanol | 50 | 50 | 50 | 40 |
| 1,3-Butyleneglycol | | | | |
| Polyethylene glycol 400 | | | | |
| Isopropyl myristate | 5 | | | |
| Diisopropyl adipate | | 5 | | 5 |
| Carboxyvinyl polymer | 1.3 | 1.3 | 1.3 | 1.3 |
| Hydroxypropylmethyl cellulose | 1.0 | 1.0 | 1.0 | 1.0 |
| Triethanolamine | | | 0.05 | 0.05 |
| Diisopropanolamine | 0.05 | 0.05 | | |
| Crotamiton | | | 5 | 5 |
| Glycol monosalicylate | | | | |
| Purified water | 17.65 | 17.65 | 27.65 | 32.65 |
| Evaluation | C | D | E | E |

C: After being kept at room temperature for one day, crystals were precipitated; D: After being kept at 5°C for one day, crystals were precipitated (when kept at room temperature, crystals were precipitated after one week); E: After being kept at 5°C for one week, crystals were precipitated (when kept at room temperature, no change was observed even after three months).

Table 2 (Continue)

| Components | No. 9 | 10 | 11 |
|---|---|---|---|
| Spironolactone | 5 | 5 | 5 |
| Ethanol | 30 | 10 | 40 |
| Isopropanol | 20 | 40 | 10 |
| 1,3-Butyleneglycol | | | |
| Polyethylene glycol 400 | | | |
| Isopropyl myristate | | | |
| Diisopropyl adipate | 5 | 5 | 5 |
| Carboxyvinyl polymer | 1.3 | 1.3 | 1.3 |
| Hydroxypropylmethyl cellulose | 1.0 | 1.0 | 1.0 |
| Triethanolamine | 0.05 | 0.05 | 0.05 |
| Diisopropanolamine | | | |
| Crotamiton | 5 | | |
| Glycol monosalicylate | | 5 | 5 |
| Purified water | 32.65 | 32.65 | 32.65 |
| Evaluation | F | G | G |

F: After being kept at 5°C for one month, crystals were precipitated (when kept at room temperature, no change was observed even after three months); G: After being kept at 5°C for one month, no change was observed (when kept at room temperature, no change was observed even after three months).

Experiment 3

In order to study the effect of crotamiton and glycol monosalicylate in a gel cream preparation, there were prepared the gel cream preparations having the formulations as shown in Table 3. As a result, as is shown in Table 3, in the preparations (No. 16 and 17) incorporated with crotamiton or glycol monosalicylate, spironolactone was completely dissolved to give a dissolved-type, homogenous gel cream preparation, but in the preparations wherein these solvents were not used (No. 12 to 15), spironolactone was not dissolved in the oily phase and its solubility was not improved even by addition of a surfactant [glycerin monostearate, polyethylene glycol (45 E.O.) monostearate], and hence, any dissolved-type gel cream preparation could not be prepared.

7

Table 3

| Components | No. 12 | 13 | 14 |
|---|---|---|---|
| Spironolactone | 5 | 5 | 5 |
| Isopropyl myristate | 30 | | |
| Diisopropyl adipate | | 30 | |
| 2-Octyldodecanol | | | 30 |
| 1,3-Butyleneglycol | 5 | 5 | 5 |
| Polyethylene glycol 400 | | | |
| Carboxyvinyl polymer | 1 | 1 | 1 |
| L-Arginine | 0.5 | 0.5 | 0.5 |
| Crotamiton | | | |
| Glycol monosalicylate | | | |
| Glycerin monostearate | 1 | 1 | 1 |
| Polyethylene glycol (45 E.O.) monostearate | 1 | 1 | 1 |
| Purified water | 56.5 | 56.5 | 56.5 |

Table 3 (continue)

| Components | No. 15 | 16 | 17 |
|---|---|---|---|
| Spironolactone | 5 | 5 | 5 |
| Isopropyl myristate | | | |
| Diisopropyl adipate | 30 | 10 | 10 |
| 2-Octyldodecanol | | | |
| 1,3-Butyleneglycol | 5 | 5 | 5 |
| Polyethylene glycol 400 | 5 | | |
| Carboxyvinyl polymer | 1 | 1 | 1 |
| L-Arginine | 0.5 | 0.5 | 0.5 |
| Crotamiton | | 5 | |
| Glycol monosalicylate | | | 5 |
| Glycerin monostearate | 1 | 1 | 1 |
| Polyethylene glycol (45 E.O.) monostearate | 1 | 1 | 1 |
| Purified water | 51.5 | 51.5 | 51.5 |

Example 1 (Ointment)

An ointment is prepared by the following formulation.

| (Components) | (Amount) |
|---|---|
| Spironolactone | 1.0 |
| Crotamiton | 5.0 |
| White vaseline | 94.0 |
| | Totally 100.0 g |

White vaseline is dissolved by warming on a water bath, and thereto is added a solution of spironolactone in crotamiton which is prepared by warming at about 70 to 80 °C, and the mixture is well stirred and continuously stirred until it becomes semisolid.

Example 2 (Ointment)

An ointment is prepared by the following formulation.

| (Components) | (Amount) |
|---|---|
| Spironolactone | 3.0 |
| Glycol monosalicylate | 10.0 |
| Diisopropyl adipate | 5.0 |
| Glycerin monostearate | 2.0 |
| White vaseline | 80.0 |
| | Totally 100.0 g |

White vaseline is dissolved by warming on a water bath, and thereto is added a solution of spironolactone in a mixture of glycol monosalicylate, diisopropyl adipate and glycerin monostearate which is prepared by warming at about 70 to 80°C, and the mixture is well stirred and continueously stirred until it becomes semisolid.

Example 3 (Ointment)

An ointment is prepared by the following formulation.

| (Components) | (Amount) |
|---|---|
| Spironolactone | 7.0 |
| Crotamiton | 5.0 |
| Glycol monosalicylate | 10.0 |
| Polyethylene glycol 400 | 40.0 |
| Polyethylene glycol 4000 | 38.0 |
| | Totally 100.0 g |

Polyethylene glycol 4000 is dissolved by warming on a water bath, and thereto is added a solution of spironolactone in a mixture of crotamiton, glycol monosalicylate and polyethylene glycol 400 which is prepared by warming at about 70 to 80°C, and the mixture is well stirred to make homogeneous.

Example 4 (Gel preparation)

A gel preparation is prepared by the following formulation.

| (Components) | (Amount) |
|---|---|
| Spironolactone | 1.0 |
| Crotamiton | 10.0 |
| Ethanol | 20.0 |
| Isopropanol | 30.0 |
| 1,3-Butyleneglycol | 5.0 |
| 4 % Carboxyvinyl polymer solution | 33.4 |
| Hydroxypropylmethyl cellulose | 0.5 |
| Triethanolamine | 0.1 |
| | Totally 100.0 g |

To a 4 % aqueous carboxyvinyl polymer solution is added triethanolamine and the mixture is made homogeneous by stirring, and thereto are added a part of ethanol, a part of isopropanol and further 1,3-butyleneglycol, and the mixture is stirred to give a homogeneous gel. To the gel is added a homogeneous mixture of a part of ethanol, a part of isopropanol and hydroxypropylmethyl cellulose. Thereto is added a previously prepared solution of spironolactone in crotamiton, and the mixture is homogeneously stirred to give a gel preparation [pH 5.3, viscosity (with type C viscometer) 50,000 cp].

9

Example 5 (Gel preparation)

A gel preparation is prepared by the following formulation.

| (Components) | (Amount) |
|---|---|
| Spironolactone | 5.0 |
| Glycol monosalicylate | 5.0 |
| Diisopropyl adipate | 5.0 |
| Ethanol | 40.0 |
| Isopropanol | 10.0 |
| 4 % Carboxyvinyl polymer solution | 32.5 |
| Hydroxypropylmethyl cellulose | 1.0 |
| Triethanolamine | 0.05 |
| Purified water | 1.45 |
| | Totally 100.0 g |

To a mixture of a 4 % aqueous carboxyvinyl polymer solution and purified water is added triethanolamine and the mixture is made homogeneous by stirring, and thereto are added a part of ethanol and a part of isopropanol, and the mixture is stirred to give a homogeneous gel. To the gel is added a homogeneous mixture of a part of ethanol, a part of isopropanol and hydroxypropylmethyl cellulose. Thereto is added a previously prepared solution of spironolactone in glycol monosalicyate and diisopropyl adipate, and the mixture is homogeneously stirred to give a gel preparation [pH 4.8, viscosity (with type C viscometer) 33,000 cp].

Example 6 (Gel preparation)

A gel preparation is prepared by the following formulation.

| (Components) | (Amount) |
|---|---|
| Spironolactone | 7.0 |
| Crotamiton | 5.0 |
| Glycol monosalicylate | 5.0 |
| Ethanol | 30.0 |
| Isopropanol | 30.0 |
| 4 % Carboxyvinyl polymer solution | 21.8 |
| Hydroxypropylmethyl cellulose | 1.0 |
| Diisopropanolamine | 0.2 |
| | Totally 100.0 g |

To a 4 % aqueous carboxyvinyl polymer solution is added diisopropanolamine and the mixture is made homogeneous by stirring, and thereto are added a part of ethanol and a part of isopropanol, and the mixture is stirred to give a homogeneous gel. To the gel is added a homogeneous mixture of a part of ethanol, a part of isopropanol and hydroxypropylmethyl cellulose. Thereto is added a previously prepared solution of spironolactone in crotamiton, glycol monosalicylate, a part of ethanol and a part of isopropanol, and the mixture is homogeneously stirred to give a gel preparation [pH 5.5, viscosity (with type C viscometer) 28,000 cp].

Example 7 (Gel cream preparation)

A gel cream preparation is prepared by the following formulation.

| (Components) | (Amount) |
|---|---|
| Spironolactone | 1.0 |
| Glycol monosalicylate | 3.0 |
| Glyceryl tri(capryl-caprate) | 10.0 |
| Diisopropyl adipate | 3.0 |
| Stearyl alcohol | 2.0 |
| Glycerin monostearate | 1.0 |
| Polyoxyl 40 monostearate | 1.0 |
| 4 % Carboxyvinyl polymer solution | 25.0 |
| 4 % L-arginine solution | 12.5 |
| 4 % disodium edetate solution | 10.0 |
| Purified water | 31.5 |
|  | Totally 100.0 g |

Spironolactone is dissolved in glycol monosalicylate, glycerin tri(capryl-caprate) and diisopropyl adipate at about 70 to 80°C. Thereto are added stearyl alcohol, glycerin monostearate and polyoxyl 40 monostearate, and the mixture is warmed to about 70 to 80°C on a water bath to make a homogeneous mixture. Separately, to a 4 % aqueous carboxyvinyl polymer solution are added a 4 % aqueous L-arginine solution, a 1 % aqueous disodium edetate solution and purified water, and the mixture is stirred to give a homogeneous gel and thereafter, the mixture is warmed at 70 to 80°C. To the mixture is added the above-prepared warmed solution of the active ingredient-containing solution. The mixture is emulsified and homogeneously mixed to give a gel cream preparation [pH 4.5, viscosity (with type C viscometer) 80,000 cp].

Example 8 (Gel cream preparation)

A gel cream preparation is prepared by the following formulation.

| (Components) | (Amount) |
|---|---|
| Spironolactone | 3.0 |
| Crotamiton | 3.0 |
| Diisopropyl adipate | 10.0 |
| Cetanol | 2.0 |
| Glycerin monostearate | 1.0 |
| Polyethylene glycol (45 E.O.) monostearate | 1.0 |
| 4 % Carboxyvinyl polymer solution | 25.0 |
| 4 % L-arginine solution | 15.0 |
| Purified water | 40.0 |
|  | Totally 100.0 g |

Spironolactone is dissolved in crotamiton and diisopropyl adipate at about 70 to 80°C. Thereto are added cetanol, glycerin monostearate and polyethylene glycol (45 E.O.) monostearate, and the mixture is warmed to about 70 to 80°C on a water both to make a homogeneous mixture. Separately, to a 4 % aqueous carboxyvinyl polymer solution are added a 4 % aqueous L-arginine solution and purified water, and the mixture is stirred to give a homogeneous gel and thereafter, the mixture is warmed at 70 to 80°C. To the mixture is added the above-prepared warmed solution of the active ingredient-containing solution. The mixture is emulsified and homogeneously mixed to give a gel cream preparation [pH 5.0, viscosity (with type C viscometer) 60,000 cp].

Effects of the Invention

In the pharmaceutical composition for the treatment of acne vulgaris of the present invention, the active spironolactone is well dissolved in the substrate for the dermatologic drug by crotamiton and/or glycol monosalicyalte, and thereby, the spironolactone is sufficiently absorbed through the skin and exhibits the desired effects.

**Claims**

1. A pharmaceutical composition for the treatment of acne vulgaris, which comprises as an active ingredient an effective amount of spironolactone, a solvent consisting of either one or both of crotamiton and glycol monosalicylate, and a conventional substrate for dermatologic drug.

2. The composition as claimed in claim 1, wherein crotamiton is contained in an amount of 0.1 to 10 parts by weight to one part by weight of spironolactone.

3. The composition as claimed in claim 1, wherein glycol monosalicylate is contained in an amount of 0.1 to 10 parts by weight to one part by weight of spironolactone.

4. The composition as claimed in any one of claims 1 to 3, wherein spironolactone is contained in an amount of 0.1 to 10 parts by weight based on the whole weight of the composition.

5. The composition as claimed in claim 4, wherein the content of spironolactone is in the range of 3 to 8 parts by weight.

6. The composition as claimed in any one of claims 1 to 4, which is in the form of any one of an ointment, a cream preparation, a gel preparation, a gel cream preparation, a lotion, or a solution.

International application No.

PCT/JP93/00675

**A. CLASSIFICATION OF SUBJECT MATTER**

Int. Cl$^5$  A61K31/585, A61K47/14, A61K47/16

According to International Patent Classification (IPC) or to both national classification and IPC

**B. FIELDS SEARCHED**

Minimum documentation searched (classification system followed by classification symbols)

Int. Cl$^5$  A61K31/585, A61K47/14, A61K47/16

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | US, A, 4543351 (Michele Messina), September 24, 1985 (24. 09. 85), Claim & IT, A, 1194185 & DE, C, 3411225 | 1-6 |
| Y | JP, A, 63-201119 (Kao Corp.), August 19, 1988 (19. 08. 88), Claim and lower column, page 2 (Family: none) | 1-6 |
| Y | JP, B1, 43-8007 (Taisho Pharmaceutical Co., Ltd.), March 27, 1968 (27. 03. 68), Claim and 4th line from the bottom, left column to line 3, right column, page 3 (Family: none) | 1-6 |

[X] Further documents are listed in the continuation of Box C.      [ ] See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| July 19, 1993 (19. 07. 93) | August 10, 1993 (10. 08. 93) |

| Name and mailing address of the ISA/ | Authorized officer |
|---|---|
| Japanese Patent Office | |
| Facsimile No. | Telephone No. |

Form PCT/ISA/210 (second sheet) (July 1992)